(19) Europäisches Patentamt
European Patent Office
Office européen des brevets

(11) **EP 2 926 868 A1**

(12) **EUROPEAN PATENT APPLICATION**

(43) Date of publication:
**07.10.2015 Bulletin 2015/41**

(51) Int Cl.:
***A61Q 19/00*** *(2006.01)*     ***A61K 8/04*** *(2006.01)*

(21) Application number: **14163100.2**

(22) Date of filing: **01.04.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Deprez, Philippe**
**17486 Girona (ES)**

(72) Inventor: **Deprez, Philippe**
**17486 Girona (ES)**

(74) Representative: **Isern-Jara, Nuria**
**Avda. Diagonal 463 Bis 2°**
**08036 Barcelona (ES)**

(54) **Composition for neutralizing chemical peelings allowing a visual control of the homogeneity and quality of the neutralization**

(57) Composition of a cream or a solution for neutralizing chemical peelings allowing a visual control of the homogeneity and quality of the neutralization are disclosed. The composition of this invention provides a satisfactory visual control in real time of the efficacy and uniformity of its neutralization, by a modification of colour that drastically limits the risks of bad - non uniform - neutralization, and the consequences of potential burning.

EP 2 926 868 A1

**Description**

BACKGROUND OF THE INVENTION

The terms "chemical peelings" commonly refer to acidic solutions intended to be applied on the skin, with the main goal of treating skin photo ageing, fine lines, pigmentations, acne, etc. Chemicals peelings are globally intended to destroy damaged skin structures and induce the skin regeneration through the production of new structures, originating from deep tissue layers, structures not damaged and having a younger aspect.

However, when the quantity of acids in the chemical peeling overpasses the skin tolerance, the skin gets burned. For this reason, shortly after their application the chemical peelings have to be neutralized using a base/alkali solution (pH higher than 7) that reacts with the acids (pH lower than 7) in order to neutralize them and avoid skin damages.

Neutralization of an acid needs the exact quantity of basic solution to reach a pH of average 7 . Any insufficiency of neutralization allows the acid substances to keep acting on the skin, potentially inducing important skin damages, transitory or definitive.

Presently, neutralization is usually done through the use of sodium bicarbonate solutions that are applied on the skin having such acids remain from the chemical peelings. Then, the following reaction occurs:

$$\text{Acid} + \text{base} = \text{salt} + \text{water}$$

Salt and water have no damaging potential to the skin.

Presently there is no possibility to check whether the neutralization has been correctly done or not, and this can lead to insufficient neutralization. Therefore, one of the objects of the present invention is to provide a solution to the problem of checking the level of neutralization of the skin as well as the uniformity of neutralization. To that end, the inventors have developed a neutralizing cream that changes of color depending on the skin acidity: when the color is uniformly blue or green, the skin is correctly neutralized and there is no longer any risk that the skin results burned. When the cream becomes yellow, the skin is not neutralized enough and more neutralizing cream should be applied.

SUMMARY OF THE INVENTION

In order to solve the above problem, the inventors have developed a solution mainly comprised a pH indicator to visualize in real time the efficacy or homogeneity of a chemical peeling neutralization composition that is applied on the skin. The pH indicator changes its color when the pH skin is around 7 and it is in a concentration between 0.0025% and 10% by weight.

DETAILED DESCRIPTION OF THE INVENTION

Medical literature describes indeed many incidents linked with bad chemical peelings neutralization. In fact, lacking of any direct control, what could be considered by a practitioner as an excess of neutralization could in reality correspond to an insufficiency. In this situation, non-neutralized acids continue reacting with the skin, burning it. Therefore, unfortunately there presently exist no possibility of controlling the quality of neutralization and that is potentially dangerous for the skin.

A control in real time of the neutralization process is therefore to be considered as a definitive progress in the world of chemical peels.

The neutralizing cream of the present invention neutralizes the acids of the peeling solution that was previously applied on the skin, using sodium carbonate, arginine and lysine.

Our research and development laboratory tried and found a simple way of visualizing in real time the efficacy of a chemical peeling neutralization.

We discovered that, introducing a color indicator in the formula of a neutralizing cream, allows an immediate visualization of the process of chemical peeling neutralization, in real time. This discovery highly reduces the risk of burnings after chemical peels.

When applied on the skin after a chemical peel, the cream of the present invention neutralizes the chemical peeling.

At the same time during which the neutralization occurs, the color of the cream changes: the cream that is naturally of blue color turns yellowish as long as pH is acidic (ph<7), turns green when the pH becomes neutral (pH=7) and keeps its blue color, when neutralization process is complete (pH>7).

A selection of a chemical pH indicator is needed for the composition of the present invention.

A pH indicator is a substance that turns its color at precise levels of acidity pH indicators are mainly and largely used in chemistry laboratories, in products for testing the acidity of pool waters, for chemical tests and in obstetrics.

No pH indicator is actually used in the field of chemical peelings, for testing the neutralization level of the peeling solutions, in real time during the process of neutralization.

We need to find a pH indicator that is soluble in water and turns its color in a range of 6 to 9. Such pH indicator can be chemical or natural.

Chemical pH indicators suitable for our purpose: Many pH indicators exist on the chemical market. We need an indicator able to change its color when the skin is at neutral pH, around pH= 7. See the table, below.

We discovered that, introducing existing pH indicators in the formula of a neutralizing cream allows a clear visualization in real time of the process of neutralization. Depending on the indicator that is used, the color of the cream is different. Chemical pH indicator (Taken out of the list above) concentration, included in our neutralizing cream, are at a concentration of 0,0025% up to 10%.

Possible pH indicators usable in the present invention:

The table below includes a description of actual several possible chemical indicators that could be used as chemical peelings neutralization indicators. New chemical indicators could be developed in the future, allowing the same kind of neutralization visualization. These new potential indicators would be considered as included in the actual patent.

| NAME | COLOR WHEN ACIDIC | CHANGE COLOR AT PH | COLOR WHEN BASIC |
|---|---|---|---|
| Bromocresol purple | yellow | 5.2-6.8 | purple |
| Bromothymol blue | yellow | 6.0-7.6 | blue |
| Phenol red | yellow | 6.4-8.0 | red |
| Neutral red | red | 6.8-8.0 | yellow |
| Naphtholphthalein | colorless to reddish | 7.3-8.7 | greenish to blue |
| Cresol Red | yellow | 7.2-8.8 | reddish-purple |

A pH indicator can be purely chemical as seen in the table but many plants or plant parts contain natural chemicals that are red in acidic solutions and blue in basic solutions.

Anthocyanins can be extracted with water or other solvents from a multitude of colored plants including red cabbage, geranium, poppy, rose petals, berries, rhubarb, *Hydrangea macrophylla,* "Litmus" was used by alchemists in the middle age as a pH indicator: red in acidic solutions, it becomes blue in alkalis solutions.

- Bromocresol purple: It is a pH indicator that turns color at pH 5,2 - 6,8. Under pH 5,2 the color is yellow, over pH 6,8, the color is purple. Bromocresol purple is used in medical laboratories to measure albumin, and also in photographic laboratories.

- Bromothymol blue: It is a pH indicator that turns its color between 6 and 7,6. Under pH 6, the product has a yellowish color. Between 6 and 7,6 the blue color becomes green: a green color shows the neutral point. Over pH 7,6 the color is blue.
  Bromothymol blue is used in chemistry, during acid-base reactions. It is also used as a pigment for stain and for managing the pH of pools and fish tanks.
  It is used during science classes to show that the more the muscles are used, the more $CO_2$ is exhaled. It is also used in some rare medical indications, as for example in obstetrics for detecting premature rupture of membranes. Amniotic fluid typically has a pH 7,2 and a specific indicator (bromothymol) will turn blue when brought in contact with fluid coming from amnion. As vaginal pH normally is acidic, the blue color indicates the presence of amniotic fluid.

- Phenol red: is a ph indicator frequently used in cell biology laboratory. Yellow when acidic, it exhibits a progressive modification of colour from yellow to red over the pH range 6.8 to 8.2. Above 8,2, phenol red turns to bright pink (fuschia). Phenol red was used in the past to estimate the overall blood flow through the kidneys. Now it is obsolete. Phenol red is actually used as an indicator for cell cultures. It has also been used as a differentiation factor in obstetrics. Phenol red is also used as pH indicator in home swimming pool test kits.

- Neutral red: is a pH indicator used for staining in histology. Red when in its acid form, it becomes yellow at pH over 8 and turns color between 6,8 and 8.

Neutral red is used as a vital stain: living cells incorporate neutral red into their lysosomes. As cells begin to die, their ability to incorporate neutral red diminishes. It is therefore used with some growth media for bacteria and cell cultures.

- Naphtolphtalein is a pH indicator. Visual transition from colorless/reddish to greenish/blue at pH 7,3-8,7.

- Cresol red: is a dye frequently used for monitoring the pH in aquaria. Under pH 7,2 the color is yellow. Over pH 8,8 the color is reddish purple. It can be used in many common molecular biology reactions, and in electrophoresis processes.

When using bromothymol blue as an indicator, for example, our neutralizing composition is naturally of blue color, it turns yellow as long as the skin is acid; green when the skin is neutral and stays blue when the acids have been completely neutralized.

For example, would some areas of the skin remain yellowish, then it would be evident that more "blue cream" should be applied, until the blue color remains stable and do not become yellowish any more.

When using neutral red, as an indicator, for example, our neutralizing cream is naturally of yellow color, it turns red when the acids have been completely neutralized.

When using phenol red as an indicator, for example, our neutralizing cream is naturally of red color, it turns yellow when the acids have been completely neutralized.

Our invention provides the practitioner a control in real time of the efficacy and uniformity of its neutralization what limits drastically the risks of bad - non uniform - neutralization, and the consequences of potential burning.

A preferred formula of neutralizer in cream is the following:

Ingredients: Water (Aqua), Sodium Carbonate, Sodium Acrylate / Sodium Acryloyldimethyl Taurate Copolymer, Isohexadecane, Arginine HCl, Lysine HCl, Phenoxyethanol, Polysorbate 80, Sorbitan Oleate, Chlorphenesin, Caprylyl Glycol, Bromothymol Blue.

As a conclusion, our invention consists in a cream for neutralizing chemical peelings, allowing a direct visual control of the quality of the neutralization, in real time, by modification of the color of the cream.

As a first aspect, the present invention is related to an aqueous formulation comprising a pH indicator to visualize in real time the efficacy or homogeneity of a chemical peeling neutralization composition that is applied on the skin or nails. The pH indicator changes its color when the pH skin is around 7 and it is in a concentration between 0.0025% and 10% by weight.

According other aspect, the pH indicator is selected from the group consisting of bromocresol purple, bromothymol blue, phenol red, neutral red, naphtolphtalein and cresol red.

Other aspect of the invention is related to a formulation that comprises the following components: water, sodium carbonate, isohexadecane, arginine HCl, lysine HCl, phenoxyethanol, polysorbate 80, sorbitan oleate, chlorphenesin, caprylyl glycol and bromothymol Blue. The formulation further comprises sodium acrylate or sodium acryloyldimethyl taurate copolymer.

Other aspect of the invention is related to a formulation that is a topic formulation that in a preferred form is a cream or a solution.

**Claims**

1. An aqueous formulation comprising a pH indicator to visualize in real time the efficacy or homogeneity of a chemical peeling neutralization composition that is applied on the skin or nails.

2. The formulation according to claim 1, wherein the pH indicator changes its color when the pH skin is around 7.

3. The formulation according to any one of claims 1 to 2, wherein the pH indicator is in a concentration between 0.0025% and 10% by weight.

4. The formulation according to any one of claims 1 to 3, wherein the pH indicator is selected from the group consisting of bromocresol purple, bromothymol blue, phenol red, neutral red, naphtolphtalein, cresol red and any indicator shifting its colour in the range of pH 6 to 9.

5. The formulation according to any one of claims 1 to 4, that comprises the following components: water, sodium carbonate, isohexadecane, arginine HCl, lysine HCl, phenoxyethanol, polysorbate 80, sorbitan oleate, chlorphenesin, caprylyl glycol and any pH indicator selected from the group consisting of bromocresol purple, bromothymol blue, phenol red, neutral red, naphtolphtalein and cresol red.

6. The formulation according to claim 5 that further comprises sodium acrylate or sodium acryloyldimethyl taurate copolymer.

7. The formulation according to any one of claims 1 to 6, wherein the formulation is a topic formulation.

8. The formulation according to any one of claims 1 to 7, wherein the formulation is a cream or a solution.

Europäisches
Patentamt

European
Patent Office

Office européen
des brevets

## EUROPEAN SEARCH REPORT

Application Number

EP 14 16 3100

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/077156 A2 (MBS S R L [IT]; DE ROSA MARIO [IT]) 27 July 2006 (2006-07-27) * page 5, line 26 - line 28; claim 1; example 7 * * page 8, line 15 - line 20 * * page 11, line 22 - line 25 * ----- | 1-8 | INV. A61Q19/00 A61K8/04 |
| A | EP 1 891 931 A1 (OREAL [FR]) 27 February 2008 (2008-02-27) * paragraphs [0013] - [0053] * ----- | 1-8 | |

TECHNICAL FIELDS
SEARCHED (IPC)

A61K
A61Q

The present search report has been drawn up for all claims

| Place of search | Date of completion of the search | Examiner |
|---|---|---|
| Munich | 25 September 2014 | Uhl, Martin |

EPO FORM 1503 03.82 (P04C01)

## ANNEX TO THE EUROPEAN SEARCH REPORT
## ON EUROPEAN PATENT APPLICATION NO.

EP 14 16 3100

This annex lists the patent family members relating to the patent documents cited in the above-mentioned European search report.
The members are as contained in the European Patent Office EDP file on
The European Patent Office is in no way liable for these particulars which are merely given for the purpose of information.

25-09-2014

| Patent document cited in search report | | Publication date | Patent family member(s) | | Publication date |
|---|---|---|---|---|---|
| WO 2006077156 | A2 | 27-07-2006 | AT | 441397 T | 15-09-2009 |
| | | | EP | 1841400 A2 | 10-10-2007 |
| | | | US | 2009196926 A1 | 06-08-2009 |
| | | | WO | 2006077156 A2 | 27-07-2006 |
| EP 1891931 | A1 | 27-02-2008 | EP | 1891931 A1 | 27-02-2008 |
| | | | FR | 2905067 A1 | 29-02-2008 |
| | | | JP | 2008050357 A | 06-03-2008 |
| | | | US | 2008200545 A1 | 21-08-2008 |

EPO FORM P0459

For more details about this annex : see Official Journal of the European Patent Office, No. 12/82